# EUROPÄISCHE PATENTSCHRIFT

(11) **EP 0 827 711 B1**
(45) Veröffentlichungstag und Bekanntmachung des Hinweises auf die Patenterteilung: **07.08.2002**
(21) Anmeldenummer: 97112208.0
(22) Anmeldetag: 17.07.1997
(51) Int. Cl.: A61B 1/00

(54) **Chirurgisches endoskopisches Gerät**
Endoscopic surgical apparatus
Appareil endoscopique chirurgical

(30) Priorität: 06.08.1996 DE 19631677
(43) Veröffentlichungstag der Anmeldung: 11.03.1998
(73) Patentinhaber: OLYMPUS WINTER & IBE GMBH, 22045 Hamburg (DE)
(72) Erfinder: Wiegand, Michael, 21509 Glinde (DE); Löffler, Monika, 22143 Hamburg (DE); Wittens, Cees H.A., Dr., 3065 DA Rotterdam (NL)
(74) Vertreter: Schaefer, Konrad, Dipl.-Phys.

(56) Entgegenhaltungen:
- WO-A-95/14425

## Beschreibung

Die Erfindung betrifft ein Gerät der im Oberbegriff des Anspruches 1 genannten Art.

Gattungsgemäße Geräte dienen zu chirurgischen Arbeiten unmittelbar unter einer Gewebeschicht, die in der Regel außen durch die Körperhaut abgedeckt ist, in einem Körperhohlraum, der natürlich oder künstlich sein kann. Das Hauptanwendungsgebiet, für das die gattungsgemäßen Geräte entwickelt wurden, sind die Arbeiten an den Perforanzvenen des menschlichen Beines Zur Erläuterung des speziellen Anwendungsgebietes solcher Geräte wird im folgenden die chirurgische endoskopische Behandlung von Perforanzvenen näher erläutert.

Das menschliche Bein wird durch ein paralleles Venensystem entsorgt. Dabei übernehmen tiefe Venen den größten Teil des abzuführenden Blutstromes. Oberflächliche Venen, die im Hausbereich laufen, tragen nur mit etwa 10 % zur Blutabfuhr bei. Zwischen den oberflächlichen und den tiefen Venen verlaufen an mehreren Stellen Querverbindungen, die sogenannten Perforanzvenen, die auf ihrem Wege nach innen Abzweigungen aufweisen.

Störungen im oberflächlichen Venensystem führen zu einer Vielzahl von Erkrankungen, die von nur kosmetisch störenden Krampfadern bis hin zu großflächigen Gewebezerstörungen reichen. Zur operativen Beseitigung dieser Probleme liegen eine Reihe von Techniken vor, bei denen z.B. nur die Perforanzvenen unterbunden werden oder die äußeren Venen völlig entfernt werden. In jedem Fall ist dabei ein Arbeiten an den Perforanzvenen erforderlich, um diese zu unterbinden oder zu durchtrennen und die getrennten Enden zu verschließen.

Offene Operationen zu solchen Arbeiten an den Perforanzvenen sind aufwendig und hinterlassen Narben. Daher setzt sich hier zunehmend endoskopische Technik durch.

Das bekannte Standardinstrumentarium zum endoskopischen Arbeiten auf diesem Gebiet, das als Vorläufer des gattungsgemäßen Gerätes angesehen werden kann, ist in dem Aufsatz
M. Jugenheimer, Th. Junginger:
"Endoscopic Subfascial Sectioning of Incompetent Perforating
Veins in Treatment of Primary Varicosis"
World J. Surg. 16, 971-975, 1992
beschrieben. Es besteht aus einem proximal offenen Schaft mit Handgriff, in den eine Optik mit Handgriff und ein Arbeitsinstrument eingeführt werden. Das Arbeiten geschieht dabei in dem Raum zwischen dem Muskel und der den Muskel umgebenden Faszie, der sich leicht durch Abheben der Faszie vom Muskel zu einem Arbeitsraum erweitern läßt.

Dieses Instrumentarium ist nachteilig, da mehrere Teile zu halten und zu betätigen sind, wozu mehr als zwei Hände erforderlich sind. Außerdem ist der Schaft offen, so daß Gasabsaugung, z.B. zum Entfernen von Rauch und Dampf bei elektrochirurgischem Arbeiten oder Gasinsufflation zum Erweitern des Arbeitstaumes nicht möglich ist.

Ein gattungsgemäßes Gerät ist in
beschrieben. Dieses Gerät weist einen Schaft mit integrierter Optik auf. Beide Teile können also von einer Hand bedient werden. Die andere Hand bleibt frei zum Bedienen des Arbeitsinstrumentes, beispielsweise einer Schere, eines Messers oder einer Hochfrequenzschneidelektrode zum Trennen der Perforanzvene, eines Koagulationsinstrumentes oder eines Clipapplikators zum Verschließen der Perforanzvene u. dgl. Durch die integrierte Konstruktion ist eine bessere Abdichtung möglich, z.B. eine am Arbeitsinstrument abdichtende Gummilippe am proximalen Ende des Arbeitskanales. Dadurch wird Insufflation und Absaugen erleichtert.

Bei dieser Konstruktion ist die Optik in dem den Abgängen benachbarten Querschnittsbereich des Schaftes angeordnet, während der Arbeitskanal den Abgängen gegenüberliegend angeordnet ist. Dadurch vereinfacht sich die Konstruktion, da bei der Führung von Bildleiter und Lichtleiter aus der Optik in die Abgänge der Arbeitskanal nicht im Wege ist.

Bei der üblichen Arbeitsweise mit einem solchen Gerät wird dieses durch einen Stichkanal unter die Haut und die Muskelfaszie in den Raum zwischen Faszie und Muskel eingeschoben und dort bis zu einer zu bearbeitenden Perforanzvene vorgeschoben, um eventuell anschließend bis zu einer weiteren in einem Arbeitsgang zu bearbeitenden benachbarten Perforanzvene weitergeschoben zu werden. Dabei liegt das Gerät naturgemäß mit seinem äußeren, aus der Haut herausragenden proximalen Ende unmittelbar am Bein an. Die Abgänge müssen aus Platzgründen und zu ihrer Handhabung vom Bein weggerichtet sein.

Daher liegt bei der bekannten Konstruktion der Arbeitskanal des Gerätes zum Beininneren hin, während die Optik zur Beinaußenseite, also zur Faszie hin, liegt. Daraus ergibt sich aber, daß ein durch den Arbeitskanal eingeschobenes Instrument, beispielsweise ein Clipapplikator in dem Raum zwischen Faszie und Muskel, in dem an der Perforanzvene gearbeitet wird, innen am Muskel anliegend liegt. Nur dort kann einigermaßen bequem an der Perforanzvene gearbeitet werden.

Andererseits ist aber das Arbeiten an dieser muskelnahen Stelle der Perforanzvene ungünstig, da die Perforanzvene in dem Arbeitsbereich, also in dem Raum zwischen Faszie und Muskel Verzweigungen aufweist. Eine Versorgung der Perforanzvene muß außerhalb dieser Verzweigungen, also möglichst weit außen an der Faszie, erfolgen.

Mit dem bekannten Gerät, bei dem das Instrument zum Muskel hin liegt, ist das Arbeiten am fasziennahen Bereich einer Perforanzvene nur sehr mühsam möglich. An dem Gerät muß dazu mit großer Kraft gehebelt werden, was mühsam ist und zu ungewünschten Verletzungen führt.

Aus der DE 29 26 919 C2 ist ein endoskopisches Gerät bekannt, bei dem in dem Rohrschaft die Optik mit dem Bildleiter zusammen mit einem getrennt verlegten Lichtleiter auf einer Seite des Querschnittes des Rohrschaftes angeordnet ist, während der Arbeitskanal auf der anderen Seite des Querschnittes liegt. Der Abgang des Bildleiters liegt wie bei der WO 95/14425 auf der Seite des Rohrschaftes, an der der Bildleiter in diesem verläuft. Der Abgang des Lichtleiters liegt jedoch, abweichend von der Konstruktion der WO 95/14425, auf der anderen Seite des Rohrschaftes, auf der der Arbeitskanal liegt. Die beiden Abgänge liegen also in bezug auf den Rohrschaft diametral gegenüber. Ersichtlich läßt sich ein solches Gerät für die vorliegenden Zwecke nicht einsetzen, da bei der erforderlichen Winkelposition mit vom Körper abgewandt liegendem Bildleiterabgang der Lichtleiterabgang unmittelbar zum Körper hin liegen würde.

Die Aufgabe der Erfindung besteht darin, ein gattungsgemäßes Gerät zu schaffen, mit dem in einem künstlich eröffneten Körperhohlraum unter einer Gewebeschicht das Arbeiten im Bereich unmittelbar an der Gewebeschicht erleichtert wird.

Diese Aufgabe wird erfindungsgemäß mit den Merkmalen des Anspruches 1 gelöst.

Bei dem erfindungsgemäßen Gerät ist die Lage von Optik und Arbeitskanal im Schaft, und zwar in bezug auf die Lage der Abgänge umgekehrt wie bei dem bekannten gattungsgemäßen Gerät. Bei der zuvor beschriebenen Arbeitslage des Gerätes, bei dem die Abgänge vom Körper weg gerichtet sind, liegt folglich das durch den Arbeitskanal eingeführte Instrument bereits konstruktionsbedingt aussen nahe der Gewebeschicht in dein Bereich, in dem z.B. im speziellen Anwendungsfall am günstigsten an der Perforanzvene gearbeitet wird. Umständliche und den Patienten traumatisierende Versuche, durch Hebeln des Schaftes in den äußeren Bereich der Perforanzvene zu kommen, können entfallen.

Vorteilhaft sind die Merkmale des Anspruches 2 vorgesehen. Auf diese bekannte Weise läßt sich die Reinigung des Gerätes verbessern und besteht die Möglichkeit, Schäfte unterschiedlichen Querschnittes je nach Bedarf zu verwenden.

Bei dem bekannten gattungsgemäßen Gerät ist die Optik zusammen mit dem Arbeitskanal in einem Innenschaft vorgesehen, den der Schaft mit geringem Abstand zur Ausbildung eines getrennten Saugkanales umgibt. Dadurch ist der Querschnitt des Arbeitskanales eingeschränkt, und es ergibt sich eine aufwendige mehrrohrige Konstruktion.

Vorteilhaft sind daher die Merkmale des Ansptuches 3 vorgesehen. Der Arbeitskanal ergibt sich dabei mit maximalem Querschnitt eingeschränkt nur in notwendiger Weise durch den Querschnitt der Optik.

Bei einer frei im Schaft liegenden Optik nach Anspruch 3 ergeben sich Justierprobleme. Leichte Fehljustierungen bei der Montage oder in der einen kuppelbaren Schaft verbindenden Trennkupplung sowie Verbiegungen der Optik bei unsachgemäßer Behandlung würden über die freie Länge der Optik zu einer Fehllage des Objektives führen und somit zu einer Verschiebung des Blickfeldes. Vorteilhaft sind daher die Merkmale des Anspruches 4 vorgesehen. Beim Ansetzen des Schaftes gleitet das distale Ende der Optik in die Führungsrinne und wird unter leichter elastischer Verbiegung der Optik in eine genau definierte Position gebracht. Die leichte distale Schrägstellung der Optik in Richtung auf den Arbeitskanal ergibt außerden eine Ausrichtung in Richtung des Arbeitsfeldes, das distal vor dem Arbeitskanal liegt. Die Blickabwinkelung im Objektiv kann daher etwas geringer gewählt werden

Vorteilhaft sind die Merkmale des Anspruches 5 vorgesehen. Ein solcher Sauganschluß kann in bekannter Weise zum Absaugen von beim Hochfrequenzschneiden auftretenden Dämpfen und auch zu einer den Arbeitsraum erweiternden Insufflation verwendet werden.

Vorteilhaft sind die Merkmale des Anspruches 6 vorgesehen. Die Anordnung aller Abgänge (für Bildleiter, Lichtleiter. Sauganschluß etc.) in derselben Winkelposition ermöglicht eine kompakte Anordnung der Abgänge, die als Handgriff benutzbar oder gestaltbar sein können und legt alle Abgänge in eine günstige Position zum Arbeiten mit dem Gerät unmittelbar am Körper.

Vorteilhaft sind die Merkmale des Anspruches 7 vorgesehen. Die Optik, die vorzugsweise in größerer Länge freitragend ausgebildet ist, wird auf diese Weise sehr stabil. Im Zwischenraum zwischen den Rohren kann das in üblicher Weise als Lichtleiter verwendete Faserbündel optisch getrennt vom Bildleiter angeordnet sein, welcher als Stablinsenoptik oder als bildübertragendes Faserbündel ausgebildet sein kann.

Dabei sind vorteilhaft die Merkmale des Anspruches 8 vorgesehen. Die Kammern ermöglichen eine hervorragende allseitige Abdichtung von Bildleiter und Lichtleiter im kritischen Bereich zwischen dem proximalen Ende der Optik und den Abgängen. Dabei besteht insbesondere auch eine gute Abdichtung zwischen Bildleiter und Lichtleiter, daß in das üblicherweise als Lichtleiter verwendete distal offene Faserbündel eindringende Feuchtigkeit nicht in den feuchtigkeitsempfindliche Linsenoberflächen aufweisenden Bildleiter eindringen kann. Die Kammern ergeben feiner einen guten mechanischen Schutz für den Bildleiter und den Lichtleiter gegen Beschädigungen beispielsweise durch in den Arbeitskanal eingeführte Arbeitsinstrumente.

In den Zeichnungen ist die Erfindung beispielsweise und schematisch dargestellt.
Es zeigen:
- Fig. 1: einen Schnitt durch eine erste Ausführungsform eines erfindungsgemäßen Gerätes in Arbeitsstellung am Operationsort.
- Fig. 2: einen Schnitt nach Linie 2 - 2 in Fig. 1.
- Fig. 3: einen vergtößerten Schnitt gemäß Fig. 1 durdch eine Ausführungsvariante des Getätes und
- Fig. 4: einen Schnitt nach Linie 4 - 4 in Fig. 3

Im Schnitt ist der Wadenmuskel 1 eines Patienten dargestellt mit darüberliegender Faszie 2 und außen abdeckendem Hautgewebe 3.

Zwischen einer äußeren Vene 4b und einer inneren Vene 4a verläuft eine Perforanzvene 4, die mit einem Clip 4c versehen werden soll. Die Perforanzvene 4 weist kurz unterhalb der Faszie 2 Abzweigungen 4d auf. Der Clip 4c soll außerhalb der Abzweigungen 4d also möglichst nahe der Faszie 2 liegen.

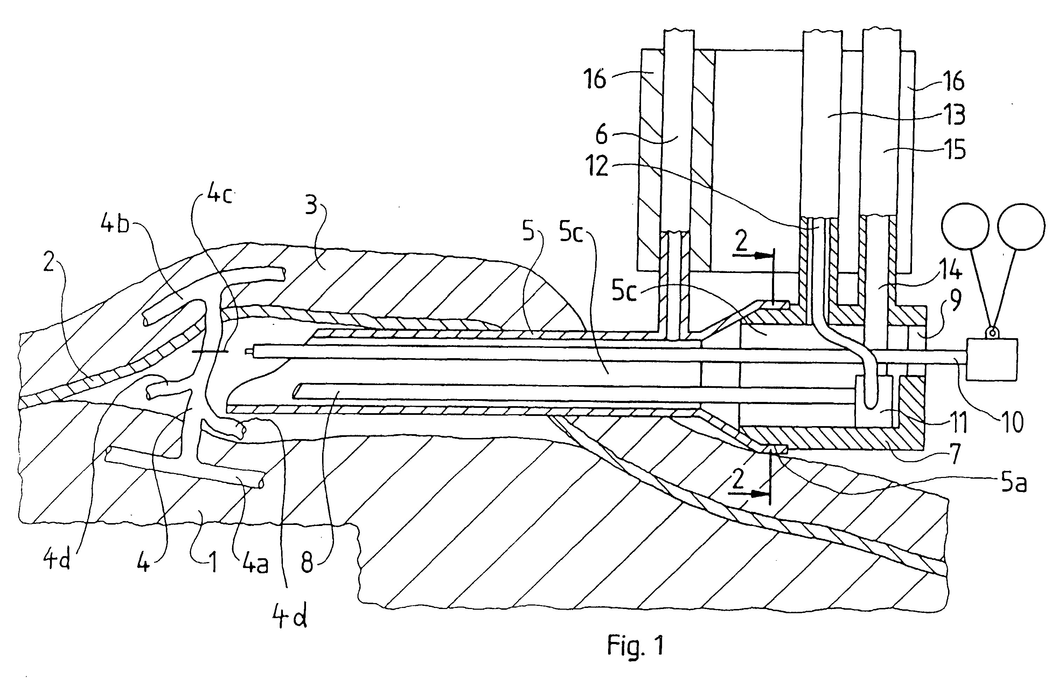

Zu diesem Zweck ist das dargestellte Gerät mit seinem als Rohr ausgebildeten Schaft 5 durch einen das Hautgewebe 3 und die Faszie 2 durchlaufenden Stichkanal in den Raum zwischen der Oberfläche des Muskels 1 und der Faszie 2 eingeführt und in den Bereich vor der Perforanzvene 4 vorgeschoben. Im proximalen Bereich des Schaftes 5, in dem dieser das Hautgewebe durchläuft, ist er vorzugsweise mit relativ großem Durchmesser ausgebildet, um in dem Stichkanal gut abzudichten. Im distalen Endbereich kann der Durclunesser verjüngt sein, obwohl ein großer Durchmesser auch hier von Vorteil ist, um den Raum zwischen Muskel 1 und Faszie 2 zu erweitern. Eine solche Erweiterung des Raumes, wie in Fig. 1 dargestellt, kann jedoch auch durch Insufflation, also durch Einblasen von Inertgas unter Druck erfolgen.

Zu diesem Zweck ist am proximalen Endbereich des Schaftes 5 ein Abgang 6 zu einem Sauganschluß vorgesehen, durch den bei Arbeiten an der Perforanzvene 4 mit Hochfrequenz auftretende Dämpfe abgesaugt werden können und der auch zur Insufflation verwendbar ist.

Über eine schematisch dargestellte, mit nicht dargestellten Mitteln verriegelbare Trennkupplung 5a ist der Schaft 5 mit einem Endstück 7 verbunden. Der Schaft 5 und das Endstück umschließen einen das gesamte Gerät durchlaufenden Arbeitskanal 5c, in dem eine stabförmige Optik 8 frei angeordnet ist, die an ihrem proximalen Ende im Endstück 7 befestigt ist.

Der Arbeitskanal 5c ist vom proximalen Ende des Endstückes 7 her mit einer Öffnung 9 zugänglich, die gegebenenfalls zur Gasabdichtung mit einer Gummimembran verschlossen sein kann, die das Durchschieben eines Arbeitsinstrumentes 10 abdichtend ermöglicht.

Das dargestellte Arbeitsinstrument 10 ist im Ausführungsbeispiel ein Clipapplikator mit Scherengriffbetätigung, der zum Setzen des Clips 4c dient. Das am distalen Ende der Optik 8 vorgesehene Objektiv ist leicht schräg blickend angeordnet, um den Arbeitsbereich am Clip 4c beobachten zu können.

Die Optik 8 ist proximal im Endstück 7 an einem Endteil 11 befestigt, von dem ein Lichtleiter 12 in üblicher Ausführung als Lichtleitfaserbündel zu einen seitlich vom Endstück 7 abgebenden Abgang 13 verläuft. Ein Bildleiter 14 verläuft zu einem Abgang 15. Der Bildleiter 14 kann als Bildleilerfaserbündel ausgebildet sein oder als Linsenoptik, beispielsweise bestehend aus Stablinsen, Umlenkprismen u. dgl.

Wie die Figuren zeigen, ist die Optik in bezug auf die Lage der Abgänge 13 und 15 im Schaft 5 gegenüberliegend angeordnet. Dies hat den Vorteil, daß der Arbeitskanal 5c den Abgängen 13 und 15 anliegend angeordnet ist. Wie Fig. 1 zeigt, liegt das Endstück 7 außerhalb des Körpers sehr nahe an der Haut des Patienten. Die Abgänge 13 und 15 ebenso wie der Abgang 6 für die Sauganschlüsse können daher nur vom Patienten weg gerichtet angeordnet sein: Ein die Abgänge 6, 13 und 15 umgebender Handgriff 16 kann zur besseren Bedienung vorgesehen sein.

Bei der sich zwangsweise ergebenden Winkellage des Gerätes gemäß Fig. 1 liegt der Arbeitskanal 5c nach außen zur Faszie 2 hin bzw. zum Hautgewebe 3, während die Optik 8 nach innen zum Muskel 1 hin liegt. Wie Fig. 1 zeigt, kann dabei der Clip 4c am äußeren Ende der Perforanzvene 4 angebracht werden unter Beobachtung durch die Optik 8, deren Bildleiter 14 und Lichtleiter 12 in nicht dargestellter Weise außerhalb des Gerätes an ein Okular oder eine Videokamera bzw. eine Lichtquelle angeschlossen sind.

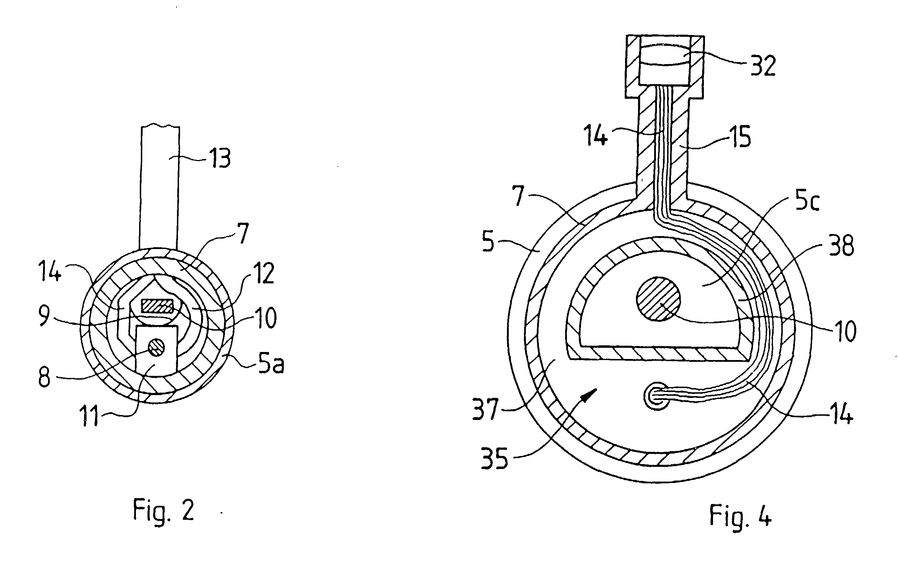

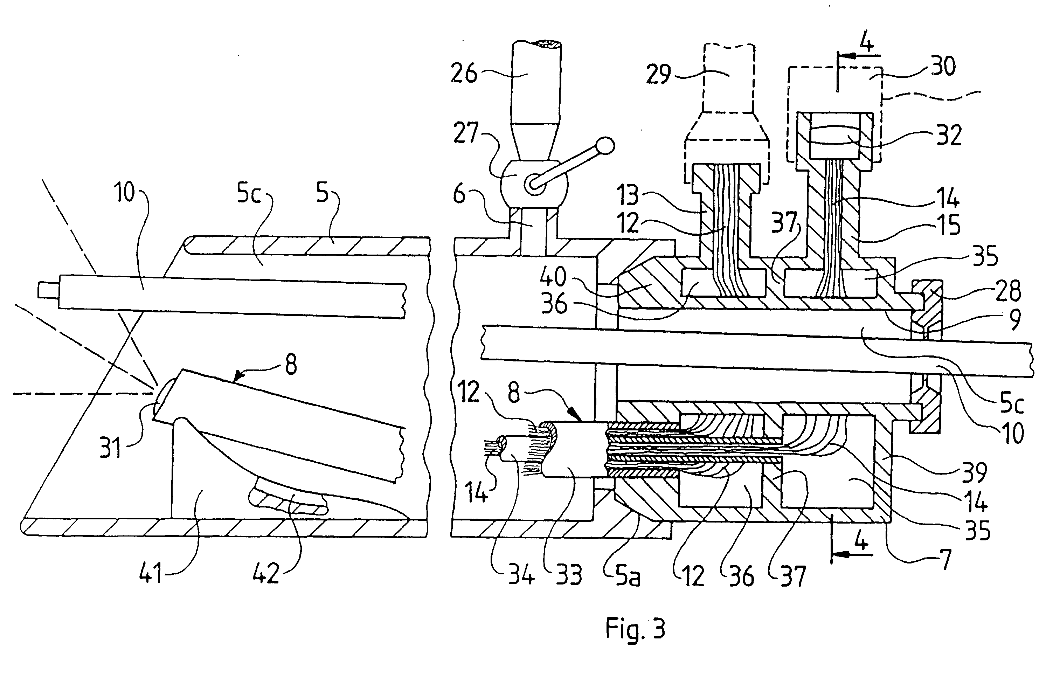

Die Fig. 3 und 4 zeigen eine Ausführungsvariante, bei der soweit wie möglich die in Fig. 1 und 2 verwendeten Bezugszeichen beibehalten wurden.

Der Schaft 5 dieses Gerätes ist wiederum über eine Trennkupplung 5a mit nicht dargestellten Verriegelungsmitteln mit dem Endstück 7 gekoppelt. Er weist einen Abgang 6 für den Sauganschluß auf mit angeschlossenem Schlauch 26 und Ventil 27

Der Arbeitskanal 5c ist proximal mit der Öffnung 9 zugänglich, an der eine elastische Membrandichtung 28 dargestellt ist, die das Arbeitsinstrument 10 abdichtend umgreifend dem Arbeitskanal 5c gasdicht verschließt.

An dein als kurzer Stutzen ausgebildeten Abgang 13 für den als Faserbündel ausgebildeten Lichtleiter 12 läßt sich ein gestrichelt dargestelltes Lichtleitkabel 29 anschließen, das zu einer nicht dargestellten Kaltlichtquelle führt. An den ebenfalls als Stutzen ausgebildeten Abgang 15 des Bildleiters 14 läßt sich eine gestrichelt dargestellte Videokamera 30 anschließen oder an deren Stelle ein Okular zur Betrachtung mit dem Auge.

Der Bildleiter 14 ist im Ausführungsbeispiel der Fig. 3 als Bildleiterbündel ausgebildet. Er fülut das Bild von der am distalen Ende der Optik 8 angeordneten Objektivlinse 31 zu einer am äußeren Ende des Abganges 15 vorgesehenen Okularlinse 32. Diese Linsen oder gegebenenfalls zusätzlich vorgesehene Fenster dichten die Enden des Bildleiters 14 gegen eindringende Flüssigkeit ab, die zum Beschlagen von Linsenoberflächen und somit zur Trübung des Bildes führen würde.

Die Optik 8 weist ein Außenrohr 33 und ein Innenrohr 34 auf. Der Bildleiter 14 ist im Inneren des Innnerohres 34 verlegt und der Lichtleiter 12 im Zwischenraum zwischen den Rohren 33 und 34.

Im Endstück 7 sind eine Bildleiterkammer 35 und eine Lichtleiterkammer 36 vorgesehen. Diese umlaufen ringförmig, wie Fig. 4 zeigt, den gegenüber den Kammern mit einem Hüllrohr 38 umgrenzten Arbeitskanal 5c. Sie sind gegeneinander mit einer Wand 37 abgegrenzt. Die Bildleiterkaminer 35 ist proximal mit einer Endwand 39 und die Lichtleiterkammer 36 distal mit einer Endwand 40 verschlossen.

Das Außenrohr 33 der Optik 8 ist in der distalen Endwand 40 gelagert, so daß sein Innenraum in die Lichtleiterkammer 36 mündet. Durch diese ist der Lichtleiter 12 bis zu seinem Abgang 13 um den Arbeitskanal 5c herum verlegt.

Das Innenrohr 34 der Optik 8 verläuft durch die Lichtleiterkammer 36 und endet in der Wand 37, die die beiden Kammern trennt, mündet also in die Bildleiterkammer 35. Durch diese ist der Bildleiter um den Arbeitskanal 5c herum bis zu seinem Abgang 15 verlegt, wie dies Fig. 4 zeigt.

Auf diese Weise lassen sich Bildleiter 14 und Lichtleiter 12 auf ihrer gesamten Länge vollständig und ohne schwierige Dichtungsprobleme sehr gut gegeneinander abgrenzen, so daß in das endseitig offene Faserbündel des Lichtleiters 12 eindringendes Wasser nicht zum Bildleiler 14 gelangen kann.

Fig. 3 zeigt, daß im distalen Endbereich des Schaftes 5 auf dessen Innenwand eine Rampe 41 angeordnet ist, die in ihrer Umfangswinkelposition in bezug auf die Lage der Abgänge 13 und 15 gegenüberliegend, also um 180° versetzt angeordnet ist. Die Rampe 41 weist eine Führungsrinne 42 auf, die vom proximalen Ende zum distalen Ende der Rampe ansteigend verläuft und mit ihrem distalen Ende im mittleren Querschnittsbereich des Schaftes 5 liegt.

Die Optik 8 ist bei der Ausführungsform der Fig. 3 bei geöffneter Trennkupplung 5a und abgenommenem Schaft 5 parallel zur Achse des Gerätes ausgerichtet, wie dies in Fig. 1 zu sehen ist. Wird der Schaft 5 über die Optik 8 bis zum Endstück 7 geschoben, um mit diesem verriegelt zu werden, so gleitet das distale Ende der Optik 8 in der Führungsrinne 42 aufwärts bis in die Endposition, die in Fig. 3 dargestellt ist. Die Optik 8 wird dabei leicht gebogen und liegt nun unter Spannung gegen das distale Ende der Führungsrinne 42 in sicherem Halt in der dargestellten Position, und zwar unabhängig von etwaigen Justierungsfehlern. Sie liegt also nach Montage des Schaftes 5 stets in ihrer Sollposition, aus der das Objektiv 31 den Arbeitsbereich erfaßt.

## Patentansprüche

1. Chirurgisches endoskopisches Gerät zum Arbeiten im Bereich unmittelbar unter einer einen Körperhohlraum nach außen abdeckenden, vom Gerät durchsetzten Gewebeschicht (2, 3), mit einem Rohrschaft (5), in dem ein zur axialen Durchführung eines Arbeitsinstrumentes (10) ausgebildeter Arbeitskanal (5c) und eine mit der Blickrichtung ihres Objektives (31) auf den Arbeitsbereich gerichtete, einen Bildleiter (14) aufweisende Optik (8) angeordnet sind, und mit einem von dem Arbeitskanal (5c) durchlaufenden proximalen Endstück (7), in dem die Optik (8) befestigt ist und aus dem der Bildleiter (14) mit einem seitlichen Abgang (15) herausgeführt ist, **dadurch gekennzeichnet, daß** der Arbeitskanal (5c) in einem dem Abgang (15) im wesentlichen benachbarten Querschnittsbereich und die Optik (8) in einem dem Abgang im wesentlichen gegenüberliegenden Querschnittsbereich des Schaftes (5) und des Endstückes (7) angeordnet sind und daß im Endstück (7) der Bildleiter (14), den Arbeitskanal (5c) seitlich umlaufend, vom proximalen Ende der Optik (8) zu dem Abgang (15) verlegt ist.

2. Gerät nach Anspruch 1, **dadurch gekennzeichnet, daß** der Schaft (5) über eine Trennkupplung (5a) abnehmbar mit dein Endstück (7) gekoppelt ist.

3. Gerät nach Anspruch 1, **dadurch gekennzeichnet, daß** die Optik (8) frei im Schaft (5) liegt, dessen restlicher Querschnitt den Arbeitskanal (5c) ausbildet.

4. Gerät nach Anspruch 3, **dadurch gekennzeichnet, daß** im distalen Endbereich des Schaftes (5) an seiner dem Abgang (15) im wesentlichen gegenüberliegenden Innenseite eine Rampe (41) mit einer in distaler Richtung von der Schaftwand bis in einen mittleren Querschnittsbereich des Schaftes (5) ansteigenden Führungsrinne (42) zur Aufnahme der Optik (8) angeordnet ist.

5. Gerät nach Anspruch 1, **dadurch gekennzeichnet, daß** im proximalen Endbereich des Schaftes (5) ein seitlicher Abgang (6) für einen Sauganschluß vorgesehen ist.

6. Gerät nach Anspruch 5, **dadurch gekennzeichnet, daß** alle vorhandenen Abgänge (6, 13, 15) in derselben Umfangswinkelposition angeordnet sind.

7. Gerät nach Anspruch 1, **dadurch gekennzeichnet, daß** die Optik (8) ein Außenrohr (33) und ein Innenrohr (34) aufweist, wobei der Bildleiter (14) im Innenrohr (34) und ein Lichtleiter (12) im Zwischenraum zwischen den Rohren (33, 34) angeordnet sind.

8. Gerät nach Anspruch 7, **dadurch gekennzeichnet, daß** im Endstück (7) zwei Kammern (35, 36) in Achsrichtung hintereinander und den Arbeitskanal (5c) seitlich umlaufend angeordnet sind, wobei der Bildleiterabgang (15) in die proximal gelegene Bildleiterkammer (35) und der Lichtleiterabgang (13) in die distal gelegene Lichtleiterkammer (36) münden und wobei das Innenrohr (34) in die Bildleiterkammer (35) und das Außenrohr (33) in die Lichtleiterkammer (36) münden

## Claims

1. A surgical endoscopic instrument designed for working in an area located immediately underneath a tissue layer (2, 3) that covers a body cavity to the exterior and that is penetrated by the instrument, comprising a tubular shaft (5) in which are arranged a working channel (5c), designed for guiding a working instrument (10), and an optical unit (8), consisting of an image guide (14) and an objective (31), oriented in its viewing direction to the working space, and comprising a proximal end piece (7) that is moved through said working channel (5c) to which said optical unit (8) is attached and where said image guide (14) comes out via a lateral outlet (15), **characterized by** said working channel (5c) being arranged in a cross sectional area located in principle in the vicinity of said outlet (15), and by said optical unit (8), being arranged in a cross sectional area of said shaft (5) and of said end piece (7) that is located in principle opposite said outlet, and by said image guide (14) being laid in said end piece (7) in lateral circumferential direction around said working channel (5c) from the proximal end of said optical unit (8) to said outlet (15).

2. An endoscopic instrument according to claim 1, **characterized by** said shaft (5) being connected to said end piece (7) by a disconnect-type coupling (5a).

3. An endoscopic instrument according to claim 1, **characterized by** said optical unit (8) lying freely in said shaft (5) and the residual cross sectional part of which forming said working channel (5c).

4. An endoscopic instrument according to claim 3, **characterized by** a ramp (41) being provided in said shaft (5) at its distal end area, in principle at its inner side opposite said outlet (15), with a guiding groove (42) rising in distal direction from the shaft wall up to a medium cross sectional area of said shaft (5) to seat said optical unit (8).

5. An endoscopic instrument according to claim 1, **characterized by** a lateral outlet (6) being provided at the proximal end area of shaft (5) for a suction connector.

6. An endoscopic instrument according to claim 5, **characterized by** all available outlets (6, 13, 15) being arranged at the same angle at circumference position.

7. An endoscopic instrument according to claim 1, **characterized by** said optical unit (8) having an outer tube (33) and an inner tube (34), with said image guide (14) being arranged in said inner tube (34) and a light guide (12) being arranged in the spacing between said tubes (33, 34).

8. An endoscopic instrument according to claim 7, **characterized by** two chambers (35, 36) being arranged in said end piece (7) in axial direction behind each other and in lateral circumferential direction around said working channel (5c), with said image guide outlet (15) leading into the proximal image guide chamber (35) and the light guide outlet (13) leading into the distal light guide chamber (36) and with said inner tube (34) leading into said image guide chamber (35) and said outer tube (33) leading into said light guide chamber (36).

## Revendications

1. Appareil chirurgical endoscopique conçu pour opérer dans une zone située immédiatement sous une couche de tissus recouvrant vers l'extérieur une cavité du corps et traversée par l'appareil, muni d'une gaine tubulaire (5) à l'intérieur de laquelle sont agencés un canal opérateur (5c) conformé de façon à permettre le passage axial d'un instrument de travail (10), et une optique (8) munie d'un conducteur pour la transmission de l'image (14) et dont l'objectif (31) est dirigé vers la zone d'intervention, et muni d'une partie d'extrémité proximale (7) traversée par le canal opérateur (5c) et dans laquelle est fixée l'optique (8) et de laquelle sort le conducteur de transmission de l'image (14) par un passage latéral (15), **caractérisé en ce que** le canal opérateur (5c) est agencé dans une zone de la section essentiellement voisine du passage (15) et l'optique (8) dans une zone de la section de la section de la gaine (5) et de la partie d'extrémité (7) essentiellement opposée à ce passage, et **en ce que**, dans la partie d'extrémité (7), le conducteur de transmission de l'image (14) est posé de l'extrémité proximale de l'optique (8) vers le passage (15) de façon à contourner latéralement le canal opérateur (5c).

2. Appareil selon la revendication 1, **caractérisé en ce que** la gaine (5) est couplée de façon amovible au moyen d'un accouplement séparable (5a) à la partie d'extrémité (7).

3. Appareil selon la revendication 1, **caractérisé en ce que** l'optique (8) est posée librement dans la gaine (5), le reste de la section de celle-ci formant le canal opérateur (5c).

4. Appareil selon la revendication 3, **caractérisé en ce qu**'est agencée dans la partie d'extrémité distale de la gaine (5), sur sa surface intérieure essentiellement opposé au passage (15), une rampe (41) munie d'une gorge de guidage (42) remontant, dans le sens distal, de la paroi de la gaine (5) jusqu'à mi-hauteur de sa section, et destinée à recevoir l'optique (8).

5. Appareil selon la revendication 1, **caractérisé en ce qu**'est prévu, dans la partie d'extrémité proximale de la gaine (5), un passage latéral (6) pour un raccord d'aspiration.

6. Appareil selon la revendication 5, **caractérisé en ce que** tous les passages disponibles (6, 13, 15) sont agencés selon la même position angulaire périphérique.

7. Appareil selon la revendication 1, **caractérisé en ce que** l'optique (8) présente un tube externe (33) et un tube interne (34), le conducteur de transmission de l'image (14) étant agencé dans le tube interne (34) et un guide de lumière (12) dans l'espace entre les tubes (33, 34).

8. Appareil selon la revendication 7, **caractérisé en ce que,** dans la partie d'extrémité (7), sont agencées l'une derrière l'autre en sens axial et à la périphérie du canal opérateur (5c) deux chambres (35, 36), le passage (15) du conducteur de transmission de l'image et le passage (13) du guide de lumière débouchant respectivement dans la chambre proximale (35) du conducteur de transmission de l'image et dans la chambre distale (36) du guide de lumière, et le tube interne (34) et le tube externe (33) débouchant respectivement dans la chambre (35) du conducteur de transmission de l'image et dans la chambre (36) du guide de lumière.
